Europäisches Patentamt
European Patent Office
Office européen des brevets

(19) (11) Veröffentlichungsnummer: **0 153 583**
**A1**

(12) **EUROPÄISCHE PATENTANMELDUNG**

(21) Anmeldenummer: 85100644.5

(22) Anmeldetag: 23.01.85

(51) Int. Cl.⁴: C 07 D 487/04
C 07 D 471/14, A 01 N 43/90

(30) Priorität: 03.02.84 DE 3403730

(43) Veröffentlichungstag der Anmeldung: 04.09.85 Patentblatt 85/36

(84) Benannte Vertragsstaaten: AT BE CH DE FR GB IT LI NL SE

(71) Anmelder: HOECHST AKTIENGESELLSCHAFT
Postfach 80 03 20
D-6230 Frankfurt am Main 80(DE)

(72) Erfinder: Schmierer, Roland, Dr.
An der Weinleite 5a
D-8901 Todtenweis(DE)

(72) Erfinder: Handte, Reinhard, Dr.
Theilweg 23
D-8901 Gablingen(DE)

(72) Erfinder: Mildenberger, Hilmar, Dr.
Fasanenstrasse 24
D-6233 Kelkheim (Taunus)(DE)

(72) Erfinder: Bauer, Klaus, Dr.
Kolpingstrasse 7
D-6054 Rodgau(DE)

(72) Erfinder: Bieringer, Hermann, Dr.
Eichenweg 26
D-6239 Eppstein/Taunus(DE)

(72) Erfinder: Bürstell, Helmut, Dr.
Am Hohlacker 65
D-6000 Frankfurt am Main 50(DE)

(54) **Isoindole, Verfahren zu ihrer Herstellung und ihre Verwendung in der Landwirtschaft.**

(57) Verbindungen der Formel

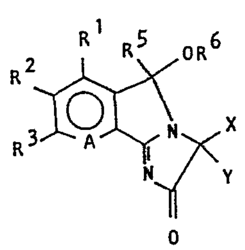

1

worin A C-$R^4$ oder N; X Alkyl, Y Alkyl, Cycloalkyl, Alkenyl, Alkinyl, Phenyl oder Benzoyl oder zusammen mit X Cycloalkyl; $R^1$ – $R^4$ H, Alkyl, Alkoxy, Alkoxycarbonyl, Halogen, $NO_2$, CN, Phenoxy oder (subst.) Phenyl; $R^5$ H oder Alkyl; $R^6$ H, (subst.) Alkyl, Halogenalkenyl, Cycloalk(en)yl, Alkinyl, (subst.) Phenyl sowie Carbonester-, Carbonamid-, Sulfoester- oder Acylreste bedeuten, sind wirksame Pflanzenbehandlungsmittel, insbesondere Herbizide.



Croydon Printing Company Ltd.

HOECHST AKTIENGESELLSCHAFT HOE 84/F 021 Dr.TG

0153583

Isoindole, Verfahren zu ihrer Herstellung und ihre Verwendung in der Landwirtschaft

Aus den DE-OS 27 00 270 und 31 21 736 sind bereits Imidazolodione bzw. Imidazolo-pyrrolo-pyridin-dione mit herbizider Wirksamkeit bekannt.

Gegenstand der vorliegenden Erfindung sind Isoindolverbindungen der Formel I

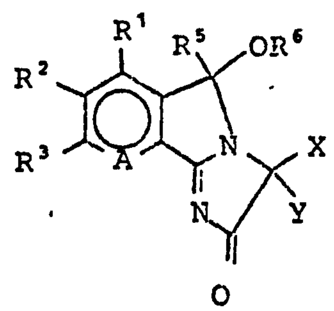

I

in welcher

| | |
|---|---|
| A | C-$R^4$ oder N |
| X | $C_1$-$C_4$-Alkyl; |
| Y | $C_1$-$C_6$-Alkyl, $C_3$-$C_6$-Cycloalkyl, $C_2$-$C_4$-Alkenyl, $C_2$-$C_4$-Alkinyl, Phenyl oder Benzyl; |
| X und Y | zusammen mit dem Kohlenstoffatom, an dem sie gebunden sind, eine gegebenenfalls durch $-CH_3$ substituierte $C_3$-$C_6$-Spirocycloalkylgruppe; |
| $R^1$, $R^2$, $R^3$ und $R^4$ | unabhängig voneinander Wasserstoff, $C_1$-$C_4$-Alkyl, $C_1$-$C_6$-Alkoxy, $C_1$-$C_6$-Alkoxycarbonyl, Halogen, Nitro, Cyano, Phenoxy oder Phenyl, das gegebenenfalls mit $C_1$-$C_4$-Alkyl, $C_1$-$C_4$-Alkoxy oder Halogen substituiert ist, wobei jeweils zwei zueinander orthoständige Reste $R^1$, $R^2$, $R^3$ oder $R^4$ gemeinsam die Gruppierung -CH=CH-CH=CH- bilden können; oder ($C_1$-$C_2$)-Halogenalkyl, insbesondere $CF_3$; |

...

$R^5$ Wasserstoff, $C_1$-$C_4$-Alkyl;

$R^6$ Wasserstoff, $C_1$-$C_{12}$-Alkyl, welches gegebenenfalls bis zu zweimal, vorzugsweise jedoch einfach mit $C_1$-$C_4$-Alkoxy, ($C_1$-$C_4$)-Alkoxyethoxy, $C_3$-$C_6$-Cycloalkyl, Benzyloxy, Phenyl, Tolyl, Halogenphenyl, Halogen, Cyano, Hydroxyl, ($C_1$-$C_4$)-Alkoxycarbonyl, Oxiranyl, Tetrahydrofuryl, ($C_1$-$C_4$)-Alkylamino, ($C_1$-$C_4$)-Dialkylamino, $C_1$-$C_4$-Alkylthio, Triazolyl, Imidazolyl oder die Gruppierung $-\overset{O}{\overset{\|}{C}}NR^7R^8$ substituiert ist; $C_3$-$C_6$-Alkenyl, $C_3$-$C_6$-Alkadienyl, $C_3$-$C_6$-Halogenalkenyl, $C_3$-$C_6$-Cycloalkyl, $C_5$ und $C_6$-Cycloalkenyl, $C_3$-$C_6$-Alkinyl; Phenyl, welches gegebenenfalls mit bis zu zwei $C_1$-$C_4$-Alkyl-, Nitro-, $C_1$-$C_4$-Alkoxycarbonyl-, Halogen- oder Methoxygruppen substituiert sein kann; $C_1$-$C_6$-Alkoxycarbonyl, Phenoxycarbonyl, Halogenphenoxycarbonyl, $C_1$-$C_4$-Alkylsulfonyl, Trihalogenmethylsulfonyl, Benzolsulfonyl, Halogenbenzolsulfonyl, Toluolsulfonyl, die Gruppierung $-\overset{O}{\overset{\|}{C}}-NR^7R^8$ oder $-\underset{O}{\underset{\|}{C}}-R^9$;

$R^7$ Wasserstoff, $C_1$-$C_4$-Alkyl;

$R^8$ Wasserstoff, $C_1$-$C_6$-Alkyl, $C_3$-$C_6$-Alkenyl, Phenyl, Halogenphenyl, Methylphenyl oder

$R^7$ und $R^8$ gemeinsam mit dem Stickstoffatom einen 5- oder 6gliedrigen Ring, in dem ein Kohlenstoffatom durch Sauerstoff oder Stickstoff ersetzt sein kann und der gegebenenfalls mit bis zu zwei Methylgruppen substituiert ist;

...

0153583

$R^9$ $(C_1-C_{12})$-Alkyl, gegebenenfalls ein- bis dreifach substituiert durch Fluor, Chlor, Brom und/oder einfach substituiert durch $(C_1-C_4)$-Alkoxy, $(C_1-C_4)$-Alkoxycarbonyl, $(C_1-C_4)$-Alkylthio, Phenyl oder Phenoxy, wobei beide gegebenenfalls bis zu dreifach durch Halogen, $(C_1-C_4)$-Alkyl, $NO_2$, $CF_3$ oder $(C_1-C_4)$-Alkoxy substituiert sein können; $(C_2-C_6)$-Alkenyl, $(C_3-C_6)$-Halogenalkenyl, $(C_3-C_6)$-Alkinyl oder Phenyl, welches ein- bis zweimal durch F, Cl, Br, $CF_3$, $(C_1-C_4)$Alkyl und/oder $(C_1-C_4)$Alkoxy substituiert sein kann bedeuten, sowie deren optische Isomeren und (für den Fall, daß A für N steht) ihre Säureadditionssalze und N-Oxide.

Steht $-NR^7R^8$ für einen heterocyclischen Ring, so handelt es sich bevorzugt um einen Pyrrolidin-, Piperidin-, Morpholin- oder N-Methyl-piperazinring.

Man erhält die erfindungsgemäßen Verbinsungen, indem man - für $R^5$ = Wasserstoff -

a) Verbindungen der allgemeinen Formel II

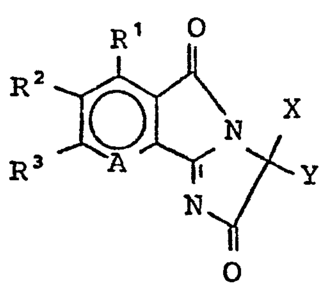

II

partiell reduziert;

b) die Dichlormethylverbindungen der Formel III

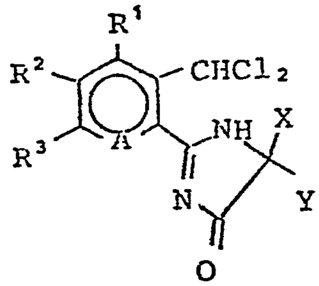

III

III

verseift; oder

c) die Dichlormethylverbindungen der Formel IV

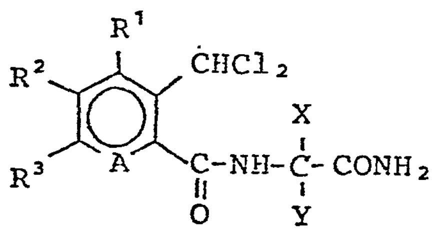

IV

IV

verseift und unter Wasserabspaltung cyclisiert; oder
- für $R^5 = C_1\text{-}C_4$-Alkyl -

d) Verbindungen der allgemeinen Formel II mit metallorganischen Alkylverbindungen umsetzt; oder
- für $R^5$ = Wasserstoff oder $C_1\text{-}C_4$-Alkyl -

e) Verbindungen der Formel V

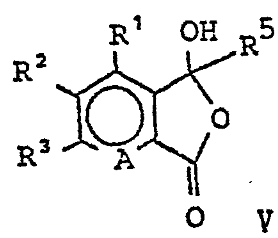

V

V

mit den Aminoamiden der Formel VI

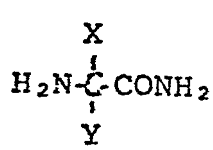

VI

VI

unter Ringschluß kondensiert und die erhaltenen Verbindungen der Formel I mit $R^6$ = Wasserstoff gewünschtenfalls durch Alkylierung, Acylierung, Sulfonierung, Carbamoylierung, Salzbildung oder Oxidation in andere Verbindungen der Formel I überführt.

zu a) Die Verbindungen II sind aus den DE-OS 31 21 736 und 27 00 270 bekannt. Zur Reduktion geeignet sind z.B. komplexe Metallhydride wie Natriumborhydrid oder desaktiviertes Lithiumalanat (vgl. z.B. Chem. Lett. 1983, 385-8).
In einem bevorzugten Verfahren wird mit Natriumborhydrid in Alkoholen oder Ethern wie Ethanol, Tetrahydrofuran oder Diethylenglykoldimethylether bei Temperaturen von -10°C bis +80°C, vorteilhaft zwischen 0 und +50°C, reduziert. Die Weiterreaktion der Isoindol-Derivate zur o-Hydroxymethylverbindung, wie sie in der DE-OS 31 21 736 beschrieben ist, tritt unter den obigen Reaktionsbedingungen überraschenderweise nicht ein.

zu b) Die Herstellung der Dichlormethylverbindungen ist in der DE-Anm. P 33 40 595.6 beschrieben. Die Verseifung erfolgt durch Umsetzung mit wäßrigen Lösungen von Alkali- oder Erdalkalihydroxyden oder -carbonaten wie Natriumhydroxid, Kaliumhydroxid oder Natriumcarbonat, bei Temperaturen von ca. 20°C bis 150°C, gegebenenfalls unter Druck. Inerte organische Lösemittel wie Toluol, Chloroform, Äthanol oder höhersiedende Äther können der Reaktion zugesetzt werden. Die Verseifung verläuft überraschend leicht und ohne daß die unter den Bedingungen zur Verseifung o-substituierter Dichlormethylverbindungen sonst stattfindende Zersetzung des Imidazolinonrings beobachtet würde.

zu c) Die Herstellung der Verbindungen IV ist ebenfalls in der DE-Anmeldung P 33 40595.6 beschrieben. Sie werden unter den in b) beschriebenen Bedingungen

verseift. Die unter Wasserabspaltung verlaufende Cyclisierung zum tricyclischen System kann schon bei der Verseifung eintreten.

zu d) Die Umsetzung der Verbindungen II mit molaren Mengen metallorganischer Alkylverbindungen, wie Lithiumalkylderivaten oder vorteilhaft Grignard-Derivaten, wird vorzugsweise bei Temperaturen von -40°C bis +40°C in Lösemitteln wie Diethylether, Ethylenglykoldimethylether, Tetrahydrofuran durchgeführt.

zu e) Die Aminoamide VI sind aus DE-OS 27 00 270 bekannt, die o-Carbonylcarbonsäuren V z.B. aus Beilstein E III, <u>10</u>, 3025. Letztere entstehen durch Tautomerisierung aus entsprechenden Aldehydsäuren:

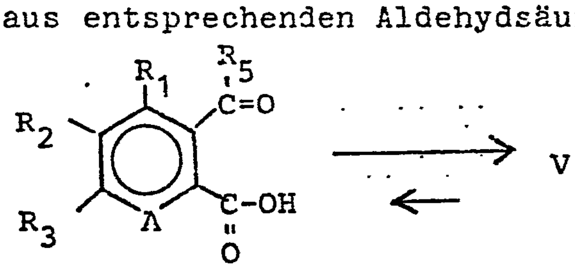

Die Kondensation von VI mit V erfolgt unter gleichzeitiger Wasserabtrennung, vorteilhaft in Gegenwart von sauren Katalysatoren wie p-Toluolsulfonsäure, bei Temperaturen von ca. 0°C bis 150°C. Das gebildete Wasser wird vorteilhaft durch Azeotropdestillation mit einem Lösemittel wie Toluol oder Xylol abgetrennt.

**Die Ausgangsstoffe der Formeln II und V können, falls $R^1 \neq R^4$ bzw. $R^2 \neq R^3$ sind, auf Grund der zu ihnen führenden Synthesewege in zwei stellungsisomeren Formen auftreten, bei denen die Substituenten $R^1$ und $R^4$ bzw. $R^2$ und $R^3$ die Plätze getauscht haben. Dieselbe Stellungsisomerie können daher auch die aus ihnen hergestellten Verbindungen der Formel I aufweisen, sodaß Gemische der jeweiligen Stellungsisomeren vorliegen können.**

Die Reaktionsprodukte der Formel I mit $R^6$ = Wasserstoff können in einfacher, an sich bekannter Weise durch Reaktion mit Alkylierungsmittel (Methyljodid, Bromessigsäuremethylester) oder Acylierungsmitteln (Säurechloride, Toluolsulfochlorid) in Gegenwart von Basen, mit Isocyanaten oder durch Oxidation, z.B. mit $H_2O_2$, zu den anderen Verbindungen der Formel I umgesetzt werden.

Die erfindungsgemäßen Verbindungen der Formel I ($R^6$ = Wasserstoff) liegen ausschließlich in der dargestellten cyclischen Halbaminalform vor.

Die vorliegenden erfindungsgemäßen Verbindungen weisen eine ausgezeichnete herbizide Wirksamkeit gegen ein breites Spektrum wirtschaftlich wichtiger mono- und dikotyler Schadpflanzen auf. Auch schwer bekämpfbare perennierende Wurzelunkräuter werden durch die Wirkstoffe gut erfaßt. Dabei ist es gleichgültig, ob die Substanzen in Vorsaat-, Vorauflauf- oder Nachauflaufspritzung ausgebracht werden. Werden die erfindungsgemäßen Verbindungen vor dem Keimen auf die Erdoberfläche appliziert, so wird das Auflaufen der Keimlinge nicht vollständig verhindert. Die Unkräuter wachsen bis zum Keimblattstadium heran, stellen jedoch dann ihr Wachstum ein und sterben schließlich nach drei Wochen vollkommen ab.

Bei Applikation der Wirkstoffe auf die grünen Pflanzenteile im Nachauflaufverfahren tritt ebenfalls sehr rasch nach der Behandlung ein drastischer Wachstumsstop ein und die Unkrautpflanzen bleiben in dem zum Applikationszeitpunkt vorhandenen Wachstumsstadium stehen oder sterben nach einer gewissen Zeit ganz ab, so daß auf diese Weise eine für die Kulturpflanzen schädliche Unkrautkonkurrenz sehr früh und nachhaltig durch den Einsatz der neuen erfindungsgemäßen Mittel beseitigt werden kann. Obgleich die

...

erfindungsgemäßen Verbindungen eine ausgezeichnete herbizide Aktivität gegenüber mono- und dikotylen Unkräutern aufweisen, werden Kulturpflanzen wirtschaftlich bedeutender Kulturen wie z. B. Weizen, Gerste, Roggen, Reis, Mais, Zuckerrübe, Baumwolle und Soja nur unwesentlich oder gar nicht geschädigt. Gegenüber dem Stand der Technik besitzen die vorliegenden erfindungsgemäßen Substanzen deshalb eine wesentlich verbesserte Selektivität bei Kulturpflanzen. Die vorliegenden Verbindungen eignen sich aus diesen Gründen sehr gut zur Bekämpfung von unerwünschtem Pflanzenwuchs in landwirtschaftlichen Nutzpflanzungen.

Darüber hinaus weisen sie wachstumsregulatorische Eigenschaften bei Kulturpflanzen auf. Sie greifen regulierend in den pflanzeneigenen Stoffwechsel ein und können damit zur Ernteerleichterung wie z. B. durch Auslösen von Desikkation, Abszission und Wuchsstauchung eingesetzt werden. Desweiteren eignen sich auch zur generellen Steuerung und Hemmung von unerwünschtem vegetativen Wachstum, ohne dabei die Pflanzen abzutöten. Eine Hemmung des vegetativen Wachstums spielt bei vielen mono- und dikotylen Kulturen eine große Rolle, da das Lagern hierdurch verringert oder völlig verhindert werden kann.

Die erfindungsgemäßen Mittel können als Spritzpulver, emulgierbare Konzentrate, versprühbare Lösungen, Stäubemittel, Beizmittel, Dispersionen, Granulate oder Mikrogranulate in den üblichen Zubereitungen angewendet werden.

Spritzpulver sind in Wasser gleichmäßig dispergierbare Präparate, die neben dem Wirkstoff außer gegebenenfalls einem Verdünnungs- oder Inertstoff noch Netzmittel, z. B. polyoxethylierte Alkylphenole, polyoxethylierte Fettalkohole, Alkyl- oder Alkylphenylsulfonate und Disper-

...

giermittel, z. B. ligninsulfonsaures Natrium, 2,2'-dinaphthylmethan-6,6'-disulfonsaures Natrium, dibutylnaphthalinsulfonsaures Natrium oder auch oleoylmethyltaurinsaures Natrium enthalten. Die Herstellung erfolgt in üblicher Weise, z. B. durch Mahlen und Vermischen der Komponenten.

Emulgierbare Konzentrate können z. B. durch Auflösen des Wirkstoffes in einem inerten organischen Lösungsmittel, z. B. Butanol, Cyclohexanon, Dimethylformamid, Xylol oder auch höhersiedenden Aromaten oder Kohlenwasserstoffen unter Zusatz von einem oder mehreren Emulgatoren hergestellt werden. Bei flüssigen Wirkstoffen kann der Lösungsmittelanteil auch ganz oder teilweise entfallen. Als Emulgatoren können beispielsweise verwendet werden. Alkyl-arylsulfonsaure Calciumsalze wie Ca-dodecylbenzolsulfonat oder nichtionische Emulgatoren wie Fettsäurepolyglykolester, Alkyl-arylpolyglykolether, Fettalkoholpolyglykolether, Propylenoxid-Ethylenoxid-Kondensationsprodukte, Fettalkohol-Propylenoxid-Ethylenoxid-Kondensationsprodukte, Alkylpolyglykolether, Sorbitanfettsäureester, Polyoxethylensorbitanfettsäureester oder Polyoxethylensorbitester.

Stäubemittel kann man durch Vermahlen des Wirkstoffes mit feinverteilten, festen Stoffen, z. B. Talkum, natürlichen Tonen wie Kaolin, Bentonit, Pyrophillit oder Diatomeenerde erhalten.

Granulate können entweder durch Verdüsen des Wirkstoffes auf adsorptionsfähiges, granuliertes Inertmaterial hergestellt werden oder durch Aufbringen von Wirkstoffkonzentrationen mittels Bindemitteln, z. B. Polyvinylalkohol, polyacrylsaurem Natrium oder auch Mineralölen auf die Oberfläche von Trägerstoffen wie Sand, Kaolinite oder von granuliertem Inertmaterial. Auch können geeig-

...

nete Wirkstoffe in der für die Herstellung von Düngemittelgranulaten üblichen Weise, gewünschtenfalls in Mischung mit Düngemitteln, granuliert werden.

In Spritzpulver beträgt die Wirkstoffkonzentration z. B. etwa 10 bis 90 Gew.-%, der Rest zu 100 Gew.-% besteht aus üblichen Formulierungsbestandteilen. Bei emulgierbaren Konzentraten kann die Wirkstoffkonzentration etwa 5 bis 80 Gew.-% betragen. Staubförmige Formulierungen enthalten meistens 5 bis 20 Gew.-% an Wirkstoff, versprühbare Lösungen etwa 2 bis 20 Gew.-%. Bei Granulaten hängt der Wirkstoffgehalt zum Teil davon ab, ob die wirksame Verbindung flüssig oder fest vorliegt und welche Granulierhilfsmittel, Füllstoffe usw. verwendet werden.

Daneben enthalten die genannten Wirkstofformulierungen gegebenenfalls die jeweils üblichen Haft-, Netz-, Dispergier-, Emulgier-, Penetrations-, Lösungsmittel, Füll- oder Trägerstoffe.

Zur Anwendung werden die in handelsüblicher Form vorliegenden Konzentrate gegebenenfalls in üblicher Weise verdünnt, z. B. bei Spritzpulvern, emulgierbaren Konzentraten, Dispersionen und teilweise auch bei Mikrogranulaten mittels Wasser. Staubförmige und granulierte Zubereitungen sowie versprühbare Lösungen werden vor der Anwendung üblicherweise nicht mehr mit weiteren inerten Stoffen verdünnt.

Mit den äußeren Bedingungen wie Temperatur, Feuchtigkeit u. a. variiert die erforderliche Aufwandmenge. Sie kann innerhalb weiter Grenzen schwanken, z. B. zwischen 0,005 und 10,0 kg/ha oder mehr Aktivsubstanz, vorzugsweise liegt sie jedoch zwischen 0,01 und 5 kg/ha.

Auch Mischungen oder Mischformulierungen mit anderen Wirkstoffen, wie z. B. Insektiziden, Akariziden, Herbi-

...

0153583

ziden, Düngemitteln, Wachstumsregulatoren oder Fungiziden sind gegebenenfalls möglich.

Nachstehend seien einige Formulierungsbeispiele aufgeführt:

Ein Stäubemittel wird erhalten, indem man 10 Gewichtsteile Wirkstoff und 90 Gewichtsteile Talkum oder Inertstoff mischt und in einer Schlagmühle zerkleinert.

Ein in Wasser leicht dispergierbares, benetzbares Pulver wird erhalten, indem man 25 Gewichtsteile Wirkstoff, 64 Gewichtsteile kaolinhaltigen Quarz als Inertstoff, 10 Gewichtsteile ligninsulfonsaures Kalium und 1 Gewichtsteil oleoylmethyltaurinsaures Natrium als Netz- und Dispergiermittel mischt und in einer Stiftmühle mahlt.

Ein in Wasser leicht dispergierbares Dispersionskonzentrat wird erhalten, indem man 20 Gewichtsteile Wirkstoff mit 6 Gewichtsteilen Alkylphenolpolyglykolether (Triton X 207), 3 Gewichtsteilen Isotridecanolpolyglykolether (8 AeO) und 71 Gewichtsteilen paraffinischem Mineralöl (Siedebereich z. B. ca. 255 bis über 377 °C) mischt und auf eine Feinheit von unter 5 Mikron vermahlt.

Ein emulgierbares Konzentrat wird erhalten aus 15 Gewichtsteilen Wirkstoff, 75 Gewichtsteilen Cyclohexanon als Lösungsmittel und 10 Gewichtsteilen oxethyliertes Nonylphenol (10 AeO) als Emulgator.

...

Die folgenden Beispiele dienen der weiteren Erläuterung der Erfindung:

A. Herstellungsbeispiele

Beispiel 1

2,3a-Dihydro-4-hydroxy-3-isopropyl-3-methyl-2-oxo-[5H]-imidazolo[1,2b][1H]isoindol

22,8 g (0,076 mol) 2-(2-Dichlormethylphenyl)-5-isopropyl-5-methyl-4-oxo-2-imidazolin werden mit 6,4 g (0,16 mol) Natriumhydroxid in 150 ml Wasser 2 h auf 90 °C erhitzt, abgekühlt und abgesaugt. Nach dem Trocknen im Vakuum verbleiben 10,5 g (56 % der Theorie) 2,3a-Dihydro-4-hydroxy-3-isopropyl-3-methyl-2-oxo-[5H]imidazolo[1,2b][1H]isoindol, ein leicht hellgelber Feststoff vom Fp 233 - 234 °C.

Beispiel 2

2,3a-Dihydro-3-isopropyl-4-methoxy-3-methyl-4-oxo-[5H]imidazolo[1,2b][1H] isoindol

10 g (0,041 mol) 2,3a-Dihydro-4-hydroxy-3-isopropyl-3-methyl-2-oxo-[5H]imidazolo[1,2b][1H]isoindol werden in 50 ml abs. DMF gelöst und bei RT mit 1,35 g (0,045 mol) 80%igem Natriumhydrid versetzt. Nachdem die Gasentwicklung beendet ist, gibt man 6,7 g (0,049 mol) Methyljodid zu, rührt 1 h bei RT nach, nimmt das Reaktionsgemisch in Toluol auf, wäscht 2mal mit Wasser, trocknet, dampft ein und erhält 9,4 g (89 % der Theorie) 2,3a-Dihydro-3-isopropyl-4-methoxy -3-methyl-4-oxo-[5H]imidazolo[1,2b][1H]isoindol, ein farbloses Öl.

...

$^1$H-NMR (60 MHz, $CDCl_3$) δ = 1,00, 1,18 (2d, J = 7 Hz, je 3H, $-CH(C\underline{H}_3)_2$); 1,67 (s, 3H, $CH_3$); 2,13 (h, J = 7 Hz, 1H, $-C\underline{H}-(CH_3)_2$); 3,18 (s, 3H, $OCH_3$) 5,65 (s, 1H, CH-O); 7,5 - 8,0 ppm (m, 4H, Phenyl).

Beispiel 3

4-Benzoyloxy-2,3 a-dihydro-3-isopropyl-3-methyl-2-oxo-[5H]imidazolo[1,2b][1H]isoindol

Analog zum Beispiel 2 wurde zu der entsprechenden Menge Natriumsalz 6,8 g (0,049 mol) Benzoylchlorid zugegeben. Nach analoger Aufarbeitung erhält man 12,7 g (89 % der Theorie) 4-Benzoyloxy-2,3a-dihydro-3-isopropyl-3-methyl-2-oxo-[5H]-imidazolo[1,2b][1H]isoindol, ein gelbes Öl.

$^1$H-NMR (60 MHz, $CDCl_3$) δ = 1,07, 1,18 (d, J = 7 Hz, je 3H, $-CH(C\underline{H}_3)_2$); 1,82 (s, 3H, $CH_3$); 2,13 (h, J = 7 Hz, 1H, $-C\underline{H}(CH_3)_2$); 6,60 (s, 1H, -CHO); 7,2 - 8,2 ppm (9H, Phenyl).

Beispiel 4

2,3a-Dihydro-4-N-methyl-carbamoyloxy-3-isopropyl-3-methyl-2-oxo-[5H]-imidazolo[1,2b][1H]isoindol

8 g (0,033 mol) 2,3a-Dihydro-4-hydroxy-3-isopropyl-3-methyl-2-oxo-[5H]imidazolo[1,2b][1H]isoindol wurden mit 2,25 g (0,04 mol) Methylisocyanat in 50 ml abs. Acetonitril 1 h bei RT gerührt. Man nimmt in 100 ml Toluol auf, wäscht 2mal mit Wasser, und trocknet über Natriumsulfat. Nach dem Einengen erhält man 7,5 g (80 % d. Theorie) 2,3a-Dihydro-4-N-methyl- ...

carbamoyloxy-3-isopropyl-3-methyl-2-oxo-[5H]-imidazolo[1,2b][1H]isoindol, ein farbloses Öl.

$^1$H-NMR (60 MHz, $CDCl_3$) $\delta$ = 1,07, 1,17 (2d, J = 7 Hz, je 3H, $CH(C\underline{H}_3)_2$); 1,73 (s, 3H, $CH_3$); 2,13 (h, J = 7 Hz, $C\underline{H}(CH_3)_2$); 2,90, 3,00 (2s, zus. 3H, $NCH_3$); 6,33 (s, 1H, CHO); 7,4 - 8,3 (m, 5H, NH, Phenyl) ppm.

In analoger Weise werden die Beispiele in Tabelle 1 hergestellt.

Tabelle 1: Verbindungen der allgemeinen Formel I

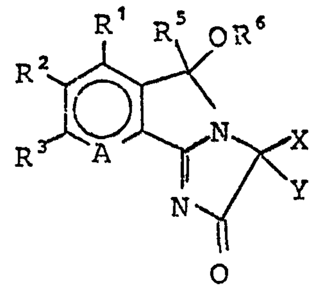

I

| Beispiel Nr. | $R^1$ | $R^2$ | $R^3$ | A | $R^4$ | $R^5$ | $R^6$ | X | Y | Fp [°C] |
|---|---|---|---|---|---|---|---|---|---|---|
| 5 | H | H | H | $C-R^4$ | H | H | H | $C_2H_5$ | $C_2H_5$ | 152-4 |
| 6 | " | " | " | " | " | " | $-CH_2-CH_2-Br$ | " | " | |
| 7 | " | " | " | " | " | " | $-CH_2-CH_2-O-C_2H_5$ | " | " | Oel |
| 8  2) | " | " | $CH_3$ | " | " | " | $-CH_3$ | $CH_3$ | $C_2H_5$ | " |
| 9 2) | " | " | " | " | " | " | 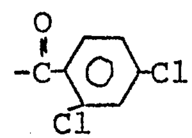 | " | $CH_3$ | " |
| 10 | " | " | H | " | " | " | $-C_2H_5$ | " | $CH(CH_3)_2$ | " |
| 11 | " | " | " | " | " | " | $-CH(CH_3)_2$ | " | " | " |
| 12 | " | " | " | " | " | " | $-n-C_6H_{13}$ | " | " | " |
| 13 | " | " | " | " | " | " | $-CH_2-CH=CH_2$ | " | " | 96-8 |

...

Fortsetzung Tabelle 1

| Beispiel Nr. | $R^1$ | $R^2$ | $R^3$ | A | $R^4$ | $R^5$ | $R^6$ | X | Y | Fp [°C] |
|---|---|---|---|---|---|---|---|---|---|---|
| 14 1) | H | H | H | $C-R^4$ | H | H | $-CH=C=CH_2$ | $CH_3$ | $CH(CH_3)_2$ | Oel |
| 15 | " | " | " | " | " | " | $-CH_2-C\equiv CH$ | " | " | 90-1 |
| 16 | " | " | " | " | " | " | $-CH_2-COOCH_3$ | " | " | 106-8 |
| 17 | " | " | " | " | " | " | 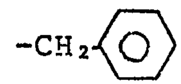 | " | " | 138-43 |
| 18 | " | " | " | " | " | " | 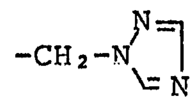 | " | " | Harz |
| 19 | " | " | " | " | " | " | 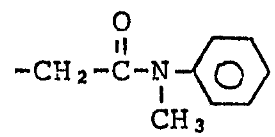 | " | " | 130-8 |
| 20 | " | " | " | " | " | " | 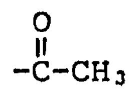 | " | " | 97-8 |
| 21 | " | " | " | " | " | " | 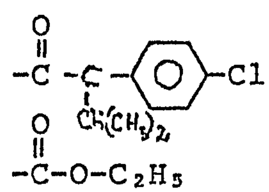 | " | " | Oel |
| 22 | " | " | " | " | " | " | | " | " | " |
| 23 | " | " | " | " | " | " | $-SO_2CH_3$ | " | " | " |

1) entsteht bei der Herstellung von 15 durch teilweise Isomerisierung

...

Fortsetzung Tabelle 1

| Beispiel Nr. | $R^1$ | $R^2$ | $R^3$ | A | $R^4$ | $R^5$ | $R^6$ | X | Y | Fp [°C] |
|---|---|---|---|---|---|---|---|---|---|---|
| 24 | H | H | H | $C-R^4$ | H | H | 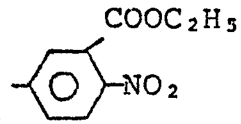 | $CH_3$ | $CH(CH_3)_2$ | |
| 25 | " | " | " | N | – | " | H | " | " | 215-20 |
| 26 | " | " | " | " | – | " | $-CH_3$ | " | " | Oel |
| 27 | " | $-CH=CH=CH=CH-$ | | " | – | " | H | " | " | |
| 28 | " | " | " | " | – | " | 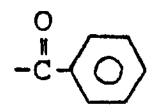 | " | " | |
| 29 2) | " | H | $CH_3$ | $C-R^4$ | H | " | H | " | " | 190-210 |
| 30 2) | Cl | " | Cl | " | " | $CH_3$ | " | " | " | |
| 31 | H | " | H | " | " | H | " | " | $-CH_2-CH(CH_3)_2$ | 148-50 |
| 32 | " | " | " | " | " | " | $-\overset{O}{\overset{\|}{C}}-CH_3$ | " | " | 75-8 |
| 33 2) | $NO_2$ | H | H | " | " | $C_2H_5$ | H | $CH_3$ | " | Oel |
| 34 | Cl | Cl | Cl | " | Cl | " | $-\overset{O}{\overset{\|}{C}}-N(CH_3)_2$ | " | " | " |
| 35 | H | $-CH=CH=CH=CH-$ | | " | H | " | 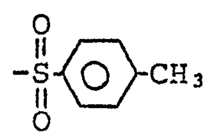 | " | " | Harz |
| 36 | " | H | H | " | " | H | H | $-CH_2-CH_2-CH_2-CH_2-\underset{CH_3}{CH}-$ | | 180-94 |

Fortsetzung Tabelle 1

| Beispiel Nr. | $R^1$ | $R^2$ | $R^3$ | A | $R^4$ | $R^5$ | $R^6$ | X | Y | Fp /°C/ |
|---|---|---|---|---|---|---|---|---|---|---|
| 37 | H | H | H | N | – | H | $-COCH_3$ | $CH_3$ | $CH(CH_3)_2$ | Öl |
| 38 | " | " | " | " | – | " | -CO- Phenyl | " | " | |
| 39 | " | " | " | " | – | " | CONH $CH_3$ | " | " | |
| 40 2) | " | " | $CH_3$ | $CR^4$ | H | " | H | " | 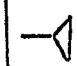 | |
| 41 2) | " | " | " | " | " | " | $SO_2CF_3$ | " | " | |
| 42 2) | " | " | " | " | " | " | $COCCl_3$ | " | " | |
| 43 2) | " | " | " | " | " | " | $CH_2-CON$ 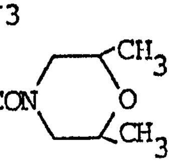 | " | " | |
| 44 2) | " | " | " | " | " | " | CON 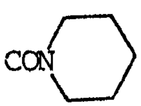 | " | " | |
| 45 2) | " | " | " | " | " | " | CONH-Phenyl | " | " | |

Fortsetzung Tabelle 1

| Beispiel Nr. | $R^1$ | $R^2$ | $R^3$ | A | $R^4$ | $R^5$ | $R^6$ | X | Y | Fp [°C] |
|---|---|---|---|---|---|---|---|---|---|---|
| 46 | H | H | H | $C-R^4$ | H | $CH_3$ | H | $CH_3$ | $-CH(CH_3)_2$ | 194-7 |
| 47 | H | H | H | N | - | H | $COCH_3$ | " | " | 113-5 |
| 48 | H | H | H | $C-R^4$ | H | H | COO Phenyl | " | " | Öl |
| 49 | H | H | H | " | H | H | CONH-Phenyl | " | " | 178-80 |
| 50 2) | $CH_3$ | H | H | " | H | H | H | " | " | 166-74 |
| 51 | H | Cl | Cl | " | H | H | H | " | " | |
| 52 | H | Cl | Cl | " | H | H | $COCH_3$ | " | " | |
| 53 2) | H | H | $CH_3$ | " | H | H | $CH_3$ | " | " | Öl |
| 54 2) | H | H | $CH_3$ | " | H | H | $COCH_3$ | " | " | Öl |
| 55 2) | H | H | $CH_3$ | " | H | H | CO-Phenyl | " | " | Öl |

2) Stellungsisomerengemisch (vgl. S.6)

## B. Biologische Beispiele

### Prüfung auf herbizide Wirkung

Die Schädigung der Unkrautpflanzen bzw. die Kulturpflanzenverträglichkeit wurde in einem Schlüssel von 0 - 5 bonitiert.

Dabei bedeutet

0 = ohne Wirkung (Schaden)
1 = 0 - 20 % Wirkung
2 = 20 - 40 % Wirkung
3 = 40 - 60 % Wirkung
4 = 60 - 80 % Wirkung
5 = 80 - 100 % Wirkung

1. Wirkung gegen Unkräuter

Samen bzw. Rhizomstücke mono- und dikotyler Unkräuter wurden in Lehmerde in Plastiktöpfen (Ø 9 cm) ausgelegt und mit Erde abgedeckt. Die als benetzbare Pulver bzw. als Emulsionskonzentrate formulierten erfindungsgemäßen Verbindungen wurden in Form wäßriger Suspensionen bzw. Emulsionen auf die Erdoberfläche appliziert. Die Wasseraufwandmenge pro Topf entsprach dabei umgerechnet 600 l/ha. Nach der Behandlung wurden die Versuchstöpfe im Gewächshaus aufgestellt und die Versuchspflanzen unter guten Wachstumsbedingungen (Temperatur: 23 ± 1 °C; rel. Luftfeuchte 60 - 80 %) kultiviert. Nach ca. 3 Wochen wurde die Pflanzenschädigung visuell bonitiert. Als Vergleich dienten dabei unbehandelte Kontrollen.

Die erfindungsgemäßen Verbindungen wiesen eine zum

...

Teil ausgezeichnete herbizide Wirksamkeit gegen wirtschaftlich bedeutende mono- und dikotyle Schadpflanzen auf (vgl. Tabelle 1).

In ähnlicher Weise werden verschiedene Unkräuter in Töpfen im Gewächshaus bis zum 3-6-Blattstadium herangezogen und dann im Nachauflaufverfahren mit den erfindungsgemäßen Verbindungen (formuliert als Spritzpulver) behandelt. 4 Wochen später wurden die Versuchspflanzen im Vergleich zu unbehandelten Kontrollpflanzen visuell bonitiert, indem die Schädigung geschätzt wurde.

Die erfindungsgemäßen Verbindungen erwiesen sich auch in diesem Versuch als gut wirksam (vgl. Tabelle 2).

Tabelle 1

Herbizide Wirkung der neuen erfindungsgemäßen Verbindungen im Vorauflauf

| Bsp.-Nr. | Dosis (kg a.i./ha) | herbizide Wirksamkeit STM | SIA | ECG | LOM |
|---|---|---|---|---|---|
| 1 | 2,5 | 5 | 5 | 5 | 5 |
| 2 | 2,5 | 5 | 5 | 2 | 2 |
| 3 | 2,5 | 5 | 5 | 5 | 5 |
| 10 | 2,5 | 5 | 5 | 4 | 4 |
| 18 | 2,5 | 5 | 5 | 2 | 1 |
| 20 | 2,5 | 5 | 5 | 5 | 5 |
| 21 | 2,5 | 4 | 5 | 1 | 2 |
| 22 | 2,5 | 4 | 5 | 2 | 4 |
| 25 | 2,5 | 5 | 5 | 5 | 5 |
| 29 | 2,5 | 5 | 5 | 5 | 5 |
| 37 | 2,5 | 5 | 5 | 5 | 4 |
| 46 | 2,5 | 5 | 5 | 4 | 4 |
| 47 | 2,5 | 5 | 5 | 1 | 5 |
| 48 | 2,5 | 5 | 5 | 5 | 4 |
| 54 | 2,5 | 5 | 5 | 4 | 3 |

Abkürzungen: STM = Stellaria media
SIA = Sinapis arvensis
LOM = Lolium multiflorum
ECG = Echinochloa crus galli
a.i. = Aktivsubstanz

Tabelle 2
Herbizide Wirkung der neuen erfindungsgemäßen Verbindungen im Nachauflauf

| Bsp.-Nr. | Dosis (kg a.i./ha) | herbizide Wirksamkeit STM | SIA | ECG | LOM |
|---|---|---|---|---|---|
| 1 | 2,5 | 5 | 5 | 2 | 5 |
| 2 | 2,5 | 3 | 5 | 4 | 2 |
| 3 | 2,5 | 3 | 5 | 3 | 2 |
| 20 | 2,5 | 5 | 5 | 3 | 5 |
| 25 | 2,5 | 5 | 5 | 5 | 5 |
| 29 | 2,5 | 4 | 5 | 4 | 5 |
| 37 | 2,5 | 5 | 5 | 4 | 5 |
| 46 | 2,5 | 4 | 5 | 2 | 2 |
| 47 | 2,5 | 4 | 5 | 1 | 1 |
| 54 | 2,5 | 2 | 5 | 3 | 3 |

Prüfung auf wachstumsregulierende Wirkung

1. Wuchshemmung bei Getreide

In Schalenversuchen im Gewächshaus wurden junge Getreidepflanzen (Weizen, Gerste und Roggen) im 3-Blattstadium mit den zu prüfenden Verbindungen tropfnaß gespritzt. Nachdem die unbehandelten Kontrollpflanzen eine Wuchshöhe von etwa 55 cm erreicht hatten, wurde bei allen Pflanzen der Zuwachs gemessen und die Wuchshemmung in % des Zuwachses der Kontrollpflanzen berechnet. Es wurde außerdem die phytotoxische Wirkung der Verbindungen beobachtet. Bei der Angabe der Wuchshemmung bedeuten 100 % den Stillstand des Wachstums und 0 % ein Wachstum entsprechend dem der unbehandelten Kontrollpflanzen.

2. Wuchshemmung an Buschbohnen

10 - 15 cm Buschbohnen werden mit den Wirkstoffzubereitungen tropfnaß bespritzt. Nach 2 Wochen wird der Zuwachs gemessen und die Wuchshemmung in % des Zuwachses der Kontrollpflanzen berechnet. Die Pflanzen weisen eine deutliche Hemmung des Längenwachstums auf.

Patentansprüche

1. Verbindungen der Formel I

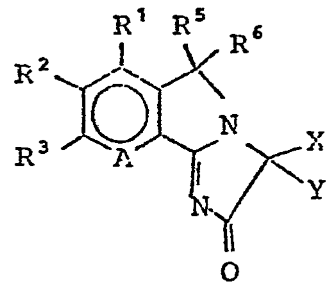

I

in welcher

A C-$R^4$ oder N

X $C_1$-$C_4$-Alkyl;

Y $C_1$-$C_6$-Alkyl, $C_3$-$C_6$-Cycloalkyl, $C_2$-$C_4$-Alkenyl, $C_2$-$C_4$-Alkinyl, Phenyl oder Benzyl;

X und Y zusammen mit dem Kohlenstoffatom, an dem sie gebunden sind, eine gegebenenfalls durch $-CH_3$ substituierte $C_3$-$C_6$-Spiro-cycloalkylgruppe;

$R^1$, $R^2$, $R^3$ und $R^4$ unabhängig voneinander Wasserstoff, $C_1$-$C_4$-Alkyl, $C_1$-$C_6$-Alkoxy, $C_1$-$C_6$-Alkoxycarbonyl, Halogen, Nitro, Cyano, Phenoxy oder Phenyl, das gegebenenfalls mit $C_1$-$C_4$-Alkyl-, $C_1$-$C_4$-Alkoxy oder Halogen substituiert ist, wobei jeweils zwei zueinander orthoständige Reste $R^1$, $R^2$, $R^3$ oder $R^4$ gemeinsam die Gruppierung -CH=CH-CH=CH-bilden können; oder $(C_1$-$C_2)$Halogenalkyl, insbesondere $CF_3$;

...

$R^5$ Wasserstoff, $C_1$-$C_4$-Alkyl;

$R^6$ Wasserstoff, $C_1$-$C_{12}$-Alkyl, welches gegebenenfalls bis zu zweimal, vorzugsweise jedoch einfach mit $C_1$-$C_4$-Alkoxy, $(C_1$-$C_4)$-Alkoxyethoxy, $C_3$-$C_6$-Cycloalkyl, Benzyloxy, Phenyl, Tolyl, Halogenphenyl, Halogen, Cyano, Hydroxyl, $(C_1$-$C_4)$-Alkoxycarbonyl, Oxiranyl, Tetrahydrofuryl, $(C_1$-$C_4)$-Alkylamino, $(C_1$-$C_4)$-Dialkylamino, $C_1$-$C_4$-Alkylthio, Triazolyl, Imidazolyl oder die Gruppierung $-\overset{O}{\overset{\|}{C}}NR^7R^8$ substituiert ist; $C_3$-$C_6$-Alkenyl, $C_3$-$C_6$-Alkadienyl, $C_3$-$C_6$-Halogenalkenyl, $C_3$-$C_6$-Cycloalkyl, $C_5$ und $C_6$-Cycloalkenyl, $C_3$-$C_6$-Alkinyl; Phenyl, welches gegebenenfalls mit bis zu zwei $C_1$-$C_4$-Alkyl-, Nitro-, $C_1$-$C_4$-Alkoxycarbonyl-, Halogen- oder Methoxygruppen substituiert sein kann; $C_1$-$C_6$-Alkoxycarbonyl, Phenoxycarbonyl, Halogenphenoxycarbonyl, $C_1$-$C_4$-Alkylsulfonyl, Trihalogenmethylsulfonyl, Benzolsulfonyl, Halogenbenzolsulfonyl, Toluolsulfonyl, die Gruppierung $-\overset{O}{\overset{\|}{C}}-NR^7R^8$ oder $-\underset{\overset{\|}{O}}{C}-R^9$;

$R^7$ Wasserstoff, $C_1$-$C_4$-Alkyl;

$R^8$ Wasserstoff, $C_1$-$C_6$-Alkyl, $C_3$-$C_6$-Alkenyl, Phenyl, Halogenphenyl, Methylphenyl oder

$R^7$ und $R^8$ gemeinsam mit dem Stickstoffatom einen 5- oder 6gliedrigen Ring, in dem ein Kohlenstoffatom durch Sauerstoff oder Stickstoff ersetzt sein kann und der gegebenenfalls mit bis zu zwei Methylgruppen substituiert ist;

$R^9$ $(C_1-C_{12})$-Alkyl, gegebenenfalls ein- bis dreifach substituiert durch Fluor, Chlor, Brom und/oder einfach substituiert durch $(C_1-C_4)$-Alkoxy, $(C_1-C_4)$-Alkoxycarbonyl, $(C_1-C_4)$-Alkylthio, Phenyl oder Phenoxy, wobei beide gegebenenfalls bis zu dreifach durch Halogen, $(C_1-C_4)$-Alkyl, $NO_2$, $CF_3$ oder $(C_1-C_4)$-Alkoxy substituiert sein können; $(C_2-C_6)$-Alkenyl, $(C_3-C_6)$-Halogenalkenyl, $(C_3-C_6)$-Alkinyl oder Phenyl, welches ein- bis zweimal durch F, Cl, Br, $CF_3$, $(C_1-C_4)$Alkyl und/oder $(C_1-C_4)$Alkoxy substituiert sein kann bedeuten, sowie deren optische Isomeren und (für den Fall, daß A für N steht) ihre Säureadditionssalze und N-Oxide.

2. Verfahren zur Herstellung von Verbindungen gemäß Anspruch 1, dadurch gekennzeichnet, daß man

- für $R^5$ = Wasserstoff -

a) Verbindungen der allgemeinen Formel II

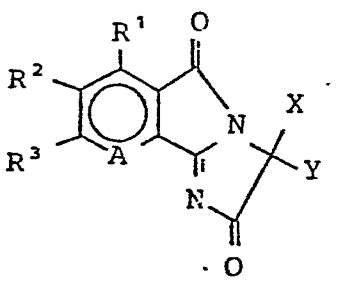

II

partiell reduziert;

b) die Dichlormethylverbindungen der Formel III

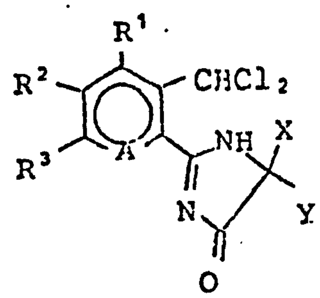

III

III

verseift; oder

c) die Dichlormethylverbindungen der Formel IV

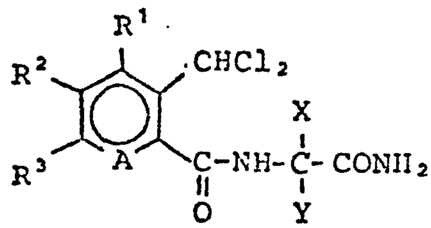

IV

IV

verseift und unter Wasserabspaltung cyclisiert; oder
- für $R^5$ = $C_1$-$C_4$-Alkyl -

d) Verbindungen der allgemeinen Formel II mit metallorganischen Alkylverbindungen umsetzt; oder
- für $R^5$ = Wasserstoff oder $C_1$-$C_4$-Alkyl -

e) Verbindungen der Formel V

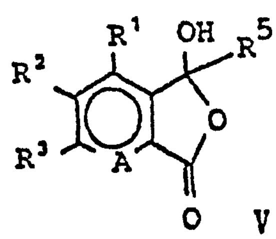

V

<u>V</u>

mit den Aminoamiden der Formel VI

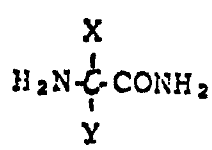

VI

<u>VI</u>

unter Ringschluß kondensiert und die erhaltenen Verbindungen der Formel I mit $R^6$ = Wasserstoff gewünschtenfalls durch Alkylierung, Acylierung, Sulfonierung, Carbamoylierung, Salzbildung oder Oxidation in andere Verbindungen der Formel I überführt.

3. Verwendung der Verbindungen gemäß Anspruch 1 als Herbizide und Wachstumsregulatoren.

4. Herbizide und wachstumsregulierende Mittel, dadurch gekennzeichnet, daß sie eine Verbindung gemäß Anspruch 1 enthalten.

5. Verfahren zur Bekämpfung von unerwünschten Pflanzen bzw. zur Wachstumsregulierung, dadurch gekennzeichnet, daß man eine wirksame Menge einer Verbindung gemäß Anspruch 1 auf die zu behandelnde Anbaufläche bzw. die zu behandelnden Pflanzen aufbringt.

6. Verbindung der Formel

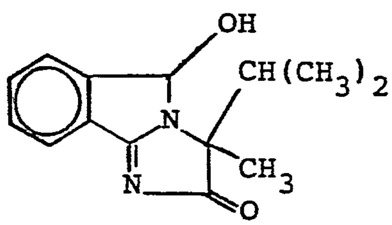

(1)

7. Verbindung der Formel

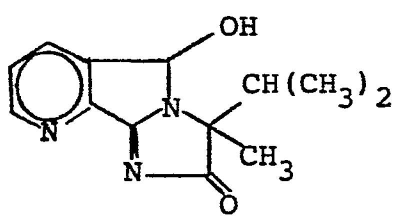

(25)

8. Verbindung der Formel

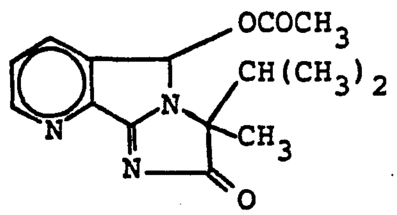

(37)

9. Verbindung der Formel

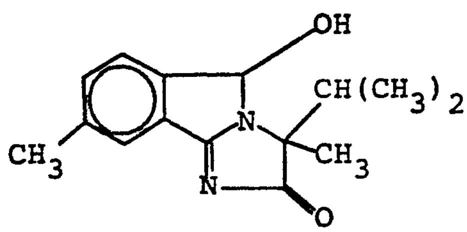

(29)

HOE 84/F 021
0153583

## Patentansprüche für Österreich:

1. Verfahren zur Herstellung von

Verbindungen der Formel I

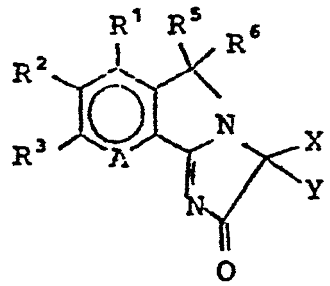

I

in welcher

| | |
|---|---|
| A | C-$R^4$ oder N |
| X | $C_1$-$C_4$-Alkyl; |
| Y | $C_1$-$C_6$-Alkyl, $C_3$-$C_6$-Cycloalkyl, $C_2$-$C_4$-Alkenyl, $C_2$-$C_4$-Alkinyl, Phenyl oder Benzyl; |
| X und Y | zusammen mit dem Kohlenstoffatom, an dem sie gebunden sind, eine gegebenenfalls durch $-CH_3$ substituierte $C_3$-$C_6$-Spiro-cycloalkylgruppe; |
| $R^1$, $R^2$, $R^3$ und $R^4$ | unabhängig voneinander Wasserstoff, $C_1$-$C_4$-Alkyl, $C_1$-$C_6$-Alkoxy, $C_1$-$C_6$-Alkoxycarbonyl, Halogen, Nitro, Cyano, Phenoxy oder Phenyl, das gegebenenfalls mit $C_1$-$C_4$-Alkyl-, $C_1$-$C_4$-Alkoxy oder Halogen substituiert ist, wobei jeweils zwei zueinander orthoständige Reste $R^1$, $R^2$, $R^3$ oder $R^4$ gemeinsam die Gruppierung $-CH=CH-CH=CH-$bilden können; oder $(C_1$-$C_2)$Halogenalkyl, insbesondere $CF_3$; |

...

$R^5$ Wasserstoff, $C_1$-$C_4$-Alkyl;

$R^6$ Wasserstoff, $C_1$-$C_{12}$-Alkyl, welches gegebenenfalls bis zu zweimal, vorzugsweise jedoch einfach mit $C_1$-$C_4$-Alkoxy, ($C_1$-$C_4$)-Alkoxyethoxy, $C_3$-$C_6$-Cycloalkyl, Benzyloxy, Phenyl, Tolyl, Halogenphenyl, Halogen, Cyano, Hydroxyl, ($C_1$-$C_4$)-Alkoxycarbonyl, Oxiranyl, Tetrahydrofuryl, ($C_1$-$C_4$)-Alkylamino, ($C_1$-$C_4$)-Dialkylamino, $C_1$-$C_4$-Alkylthio, Triazolyl, Imidazolyl oder die Gruppierung $-\overset{O}{\overset{\|}{C}}NR^7R^8$ substituiert ist; $C_3$-$C_6$-Alkenyl, $C_3$-$C_6$-Alkadienyl, $C_3$-$C_6$-Halogenalkenyl, $C_3$-$C_6$-Cycloalkyl, $C_5$ und $C_6$-Cycloalkenyl, $C_3$-$C_6$-Alkinyl; Phenyl, welches gegebenenfalls mit bis zu zwei $C_1$-$C_4$-Alkyl-, Nitro-, $C_1$-$C_4$-Alkoxycarbonyl-, Halogen- oder Methoxygruppen substituiert sein kann; $C_1$-$C_6$-Alkoxycarbonyl, Phenoxycarbonyl, Halogenphenoxycarbonyl, $C_1$-$C_4$-Alkylsulfonyl, Trihalogenmethylsulfonyl, Benzolsulfonyl, Halogenbenzolsulfonyl, Toluolsulfonyl, die Gruppierung $-\overset{O}{\overset{\|}{C}}-NR^7R^8$ oder $-\underset{\underset{O}{\|}}{C}-R^9$;

$R^7$ Wasserstoff, $C_1$-$C_4$-Alkyl;

$R^8$ Wasserstoff, $C_1$-$C_6$-Alkyl, $C_3$-$C_6$-Alkenyl, Phenyl, Halogenphenyl, Methylphenyl oder

$R^7$ und $R^8$ gemeinsam mit dem Stickstoffatom einen 5- oder 6gliedrigen Ring, in dem ein Kohlenstoffatom durch Sauerstoff oder Stickstoff ersetzt sein kann und der gegebenenfalls mit bis zu zwei Methylgruppen substituiert ist;

$R^9$ $(C_1-C_{12})$-Alkyl, gegebenenfalls ein- bis dreifach substituiert durch Fluor, Chlor, Brom und/oder einfach substituiert durch $(C_1-C_4)$-Alkoxy, $(C_1-C_4)$-Alkoxycarbonyl, $(C_1-C_4)$-Alkylthio, Phenyl oder Phenoxy, wobei beide gegebenenfalls bis zu dreifach durch Halogen, $(C_1-C_4)$-Alkyl, $NO_2$, $CF_3$ oder $(C_1-C_4)$-Alkoxy substituiert sein können; $(C_2-C_6)$-Alkenyl, $(C_3-C_6)$-Halogenalkenyl, $(C_3-C_6)$-Alkinyl oder Phenyl, welches ein- bis zweimal durch F, Cl, Br, $CF_3$, $(C_1-C_4)$Alkyl und/oder $(C_1-C_4)$Alkoxy substituiert sein kann bedeuten, sowie deren optische Isomeren und (für den Fall, daß A für N steht) ihre Säureadditionssalze und N-Oxide.

dadurch gekennzeichnet, daß man

- für $R^5$ = Wasserstoff -

a) Verbindungen der allgemeinen Formel II

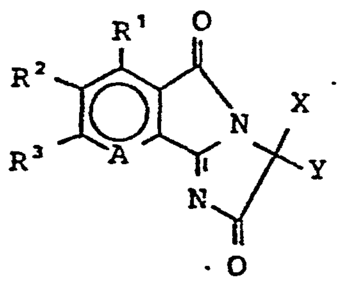

II

partiell reduziert;

b) die Dichlormethylverbindungen der Formel III

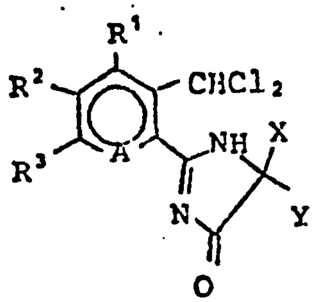

III

III

verseift; oder

c) die Dichlormethylverbindungen der Formel IV

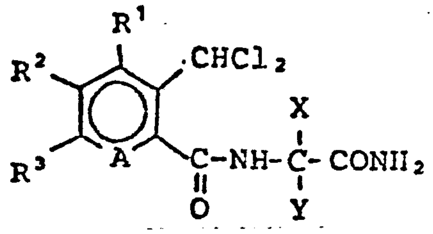

IV

IV

verseift und unter Wasserabspaltung cyclisiert; oder – für $R^5 = C_1$-$C_4$-Alkyl –

d) Verbindungen der allgemeinen Formel II mit metallorganischen Alkylverbindungen umsetzt; oder – für $R^5$ = Wasserstoff oder $C_1$-$C_4$-Alkyl –

e) Verbindungen der Formel V

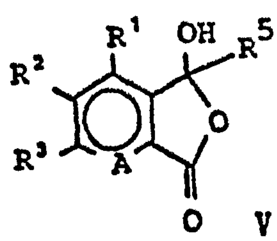

V

<u>V</u>

mit den Aminoamiden der Formel VI

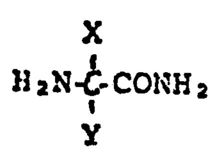

VI

<u>VI</u>

unter Ringschluß kondensiert und die erhaltenen Verbindungen der Formel I mit $R^6$ = Wasserstoff gewünschtenfalls durch Alkylierung, Acylierung, Sulfonierung, Carbamoylierung, Salzbildung oder Oxidation in andere Verbindungen der Formel I überführt.

2. Verwendung der Verbindungen gemäß Anspruch 1 als Herbizide und Wachstumsregulatoren.

3. Herbizide und wachstumsregulierende Mittel, dadurch gekennzeichnet, daß sie eine Verbindung gemäß Anspruch 1 enthalten.

4. Verfahren zur Bekämpfung von unerwünschten Pflanzen bzw. zur Wachstumsregulierung, dadurch gekennzeichnet, daß man eine wirksame Menge einer Verbindung gemäß Anspruch 1 auf die zu behandelnde Anbaufläche bzw. die zu behandelnden Pflanzen aufbringt.

Europäisches Patentamt

**EUROPÄISCHER RECHERCHENBERICHT**

0153583 Nummer der Anmeldung

EP 85100644.5

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int Cl 4) |
|---|---|---|---|
| D,A | DE - A1 - 2 700 270 (AMERICAN CYANAMID CO.)<br>* Ansprüche 1,11,14,15 *<br>-- | 1-9 | C 07 D 487/04<br>C 07 D 471/14<br>A 01 N 43/90 |
| A | DE - A1 - 2 700 269 (AMERICAN CYANAMID CO.)<br>* Ansprüche 1,11,16 *<br>-- | 1-9 | |
| A | EP - A2 - 0 041 623 (AMERICAN CYANAMID COMPANY)<br>* Ansprüche 1,2,4; Seiten 9,10,20-23 *<br>-- | 1-9 | |
| A | DE - A - 2 106 694 (AMERICAN HOME PRODUCTS CORP.)<br>* Ansprüche 1,23 *<br>-- | 1,2,6-9 | RECHERCHIERTE SACHGEBIETE (Int. Cl.4) |
| A | DE - A - 1 695 228 (F. HOFFMANN - LA ROCHE & CO. AG)<br>* Ansprüche 1,3 *<br>---- | 1,2,6-9 | C 07 D 487/00<br>C 07 D 471/00 |

Der vorliegende Recherchenbericht wurde für alle Patentansprüche erstellt.

| Recherchenort | Abschlußdatum der Recherche | Prüfer |
|---|---|---|
| WIEN | 09-05-1985 | PETROUSEK |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503. 03 82